# EUROPEAN PATENT APPLICATION

(11) **EP 4 140 430 A1**
(43) Date of publication of application: **01.03.2023**
(21) Application number: 22191547.3
(22) Date of filing: 22.08.2022
(51) Int. Cl.: A61B 18/14, A61B 18/00, A61B 90/98

(54) **ACTIVE CONTROL OF SURGICAL SMOKE EVACUATION**

(30) Priority: 24.08.2021 US 202163236377 P; 20.07.2022 US 202217868851
(71) Applicant: Covidien LP, Mansfield, MA 02048 (US)
(72) Inventor: JOSEPH, Daniel, Boulder, 80301 (US); VALENTINE, Christopher A., Boulder, 80301 (US)
(74) Representative: Maschio, Antonio

(57) **Abstract**

An electrosurgical smoke evacuation pencil includes a handle housing having a proximal end portion and a distal end portion, the handle housing defining a lumen therethrough, a nozzle in fluid communication with the lumen, a pressure sensor configured to sense a vacuum pressure at the nozzle, a connector coupled to the distal end portion of the handle housing, the connector configured to couple to a suction source, and an electrode configured to couple to a source of electrosurgical energy.

## Description

### CROSS-REFERENCE TO RELATED APPLICATIONS

The present application claims the benefit of and priority to U.S. Provisional Patent Application Serial No. 63/236,377, filed on August 24, 2021, the entire content of which being hereby incorporated by reference.

### Technical Field

The disclosure relates to surgical devices. More specifically, the disclosure relates to active control of handheld smoke evacuation electrosurgical pencils.

### Background of Related Art

Electrosurgical (ES) pencils are used in surgery, typically for cutting tissue and/or for coagulating blood vessels. An ES pencil usually includes a handpiece into which electrodes of various shapes and sizes may be placed. The ES pencil is coupled to an ES generator, such as Medtronic's Valleylab^{™} LS10 or FT10 generator, which supplies the electrode with a high frequency, typically radio frequency (RF) alternating current. The ES generator may supply various waveforms suitable for achieving various surgical effects, such as cutting, coagulating, blending, spraying, fulgurating, and the like.

While using an ES pencil, smoke is often generated. An effective way to evacuate surgical smoke is to use an ES pencil with an integrated smoke evacuation nozzle in conjunction with a suction device and an ultra-low penetration air (ULPA) filter. Conventional ES pencils rely on smoke evacuation shrouds attached to the ES pencil, which suction the smoke away via a suction device. Smoke shrouds are available either as an integrated part of the ES pencil or as a separate shroud attached to the ES pencil. A smoke nozzle, situated near the pencil's electrode, draws the smoke plume into and through the pencil's body, through a long flexible hose, and finally into a powered suction device outside of the surgical field.

During a surgical procedure, it is often desirable to use a higher vacuum pressure to evacuate more smoke. However, this higher vacuum pressure may undesirably mechanically suck tissue into the inlet of the suction conduit if it comes in contact with the tissue.

### SUMMARY

The disclosure provides an electrosurgical (ES) pencil having a handle housing and a smoke nozzle, which may be removable, integrated, and/or telescopic relative to a handle housing. The ES pencil includes an electrical plug configured to couple to an electrosurgical generator. The handle housing may have an ergonomic shape and have a slim cross-sectional area (e.g., having a height, width, or diameter of from about 10 mm to about 20 mm). The nozzle evacuates surgical smoke through the handle housing and through smoke evacuation tubing into a smoke evacuator. The smoke evacuation tubing may be corrugated to minimize kinking and to allow for free and natural movement of the ES pencil. The nozzle may be clear to aid with visualization of an electrode and its electrode tip. The nozzle also directs the smoke past a printed circuit board (PCB) coupled to a rocker switch to limit alternative current paths due to smoke intrusion into sensitive electronic components. The PCB may have an over mold on the front and back of the pencil, as well as tape that covers the PCB to limit moisture ingress. A rocker switch may be disposed over the PCB and used to control the energy delivered by the ES pencil by engaging the pushbutton switches disposed on the PCB.

The disclosure includes multiple embodiments, each of which includes multiple aspects. Various aspects of the embodiments are interchangeable among the disclosed aspects.

In accordance with embodiments of the disclosure, an ES smoke evacuation pencil includes a handle housing having a proximal end portion and a distal end portion, the handle housing defining a lumen therethrough, a nozzle in fluid communication with the lumen, a pressure sensor configured to sense a vacuum pressure at the nozzle, a connector coupled to the distal end portion of the handle housing, the connector configured to couple to a suction source, and an electrode configured to couple to a source of electrosurgical energy.

In an aspect of the disclosure, the ES pencil may further include a valve to reduce or remove the vacuum pressure at the nozzle when actuated.

In another aspect of the disclosure, the valve may be actuated to an open position in response to a pressure signal measured by the pressure sensor exceeding a preset threshold.

In yet another aspect of the disclosure, the pressure sensor may be integrated into the valve.

In a further aspect of the disclosure, the ES pencil may further include a motion sensor disposed on the nozzle. The motion sensor may transmit a motion signal to the suction source to initiate suction.

In yet a further aspect of the disclosure, the valve may include a cover to prevent actuation of the valve by an external force.

In an aspect of the disclosure, the connector may include a mating sensor to sense a mating connection with the suction source.

In another aspect of the disclosure, the active sensor may include a unique identifier.

In yet another aspect of the disclosure, the connector may be configured for electrical communication between the pressure sensor and the suction source.

In a further aspect of the disclosure, the connector may include a mechanical interface to mate with a suction conduit having a counterpart mechanical interface.

In accordance with aspects of the disclosure, a surgical smoke evacuation system includes a smoke evacuator including a suction generator configured to create a vacuum pressure, an ES pencil, a suction conduit coupling a nozzle to the smoke evacuator, a valve configured to reduce the vacuum pressure from the nozzle of the ES pencil, a processor, and a memory. The ES pencil further includes a pressure sensor configured to sense vacuum pressure at the nozzle. The memory includes instructions stored thereon, which, when executed by the processor, cause the surgical smoke evacuation system to sense the vacuum pressure at the nozzle and actuate the valve to an open position based on the sensed pressure.

In an aspect of the disclosure, the valve may open to reduce the vacuum pressure from the nozzle of the ES pencil in response to the sensed vacuum force being greater than a predetermined threshold.

In another aspect of the disclosure, the smoke evacuation system may further include a motion sensor disposed on the nozzle. The instructions, when executed by the processor, may further cause the system to sense a signal by the motion sensor indicating movement of the ES pencil and initiate suction by the smoke evacuator based on the sensed signal.

In yet another aspect of the disclosure, the smoke evacuation system may further include a motion sensor disposed on the nozzle. The instructions, when executed by the processor, may further cause the system to sense a signal by the motion sensor indicating that the ES pencil is at a resting position and stop suction by the smoke evacuator based on the sensed signal.

In a further aspect of the disclosure, the valve may include a cover to prevent actuation of the valve by an external force.

In yet a further aspect of the disclosure, the pressure sensor may be integrated into the valve.

In an aspect of the disclosure, the instructions, when executed by the processor, may further cause the system to dynamically adjust the vacuum pressure generated by the smoke evacuator based on the sensed vacuum pressure at the nozzle.

In another aspect of the disclosure, the instructions, when executed by the processor, may further cause the system to determine the nozzle is occluded based on the sensed signal and dynamically adjust the vacuum pressure generated by the smoke evacuator based on the determined occlusion.

In yet another aspect of the disclosure, the instructions, when executed by the processor, may further cause the system to determine whether the occlusion is removed based on the sensed signal and dynamically increase the vacuum pressure generated by the smoke evacuator based on the determination the occlusion was removed.

In a further aspect of the disclosure, the ES pencil may include a connector to couple to a suction source, where the connector includes a mating sensor to sense a mating connection with the suction source

### BRIEF DESCRIPTION OF THE DRAWINGS

Aspects of the disclosure are described herein with reference to the accompanying drawings, wherein:
FIG. 1 is a perspective view of a surgical smoke evacuation system, in accordance with the disclosure;
FIG. 2 is a perspective view of a smoke evacuation electrosurgical (ES) pencil of the surgical smoke evacuation system of FIG. 1, in accordance with the disclosure;
FIG. 3 is a perspective, cross-sectional view of the ES pencil of the surgical smoke evacuation system of FIG. 1 with a nozzle extender attachment according to the disclosure;
FIGS. 4 and 5 re side, cross-sectional, schematic views of the ES pencil with a valve cover and the surgical smoke evacuation system of FIG. 1 according to yet another aspect of the disclosure;
FIG. 6 is a side, cross-sectional, schematic view of the surgical smoke evacuation system of FIG. 1 with a flapper bleed valve according to another aspect of the disclosure; and
FIGS. 7 and 8 are side, cross-sectional, schematic views of the surgical smoke evacuation system of FIG. 1, with the valve located at a smoke according to yet another aspect of the disclosure.

### DETAILED DESCRIPTION OF ASPECTS

Embodiments of the disclosure are now described in detail with reference to the drawings in which like reference numerals designate identical or corresponding elements in each of the drawings. The aspects may be combined in any manner consistent with the functionality of the apparatus and/or method disclosed herein. As used herein, the term "clinician" refers to a doctor, a nurse or any other care provider and may include support personnel. Throughout this description, the term "proximal" will refer to the portion of the device or component thereof that is closer to the clinician and the term "distal" will refer to the portion of the device or component thereof that is farther from the clinician. The terms "substantially equal to" or "substantially the same" denote values that are within ±5% of each other. Additionally, in the drawings and in the description that follows, terms such as front, rear, upper, lower, top, bottom, and similar directional terms are used simply for convenience of description and are not intended to limit the disclosure. In the following description, well-known functions or constructions are not described in detail to avoid obscuring the disclosure in unnecessary detail.

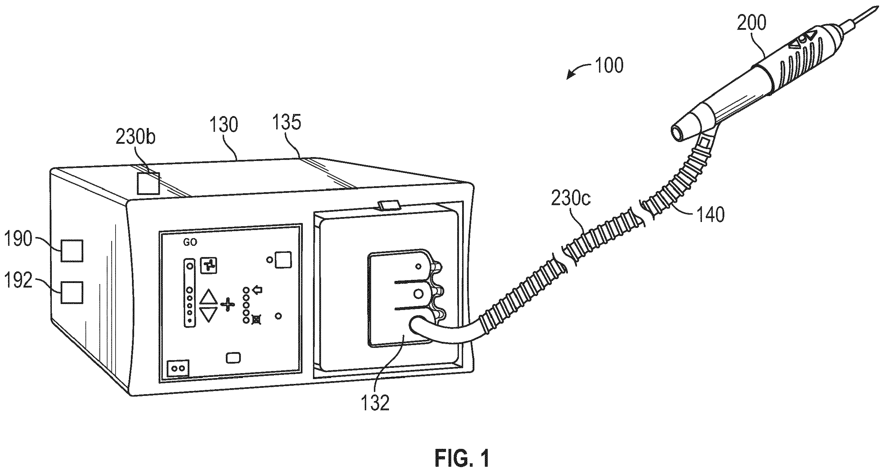

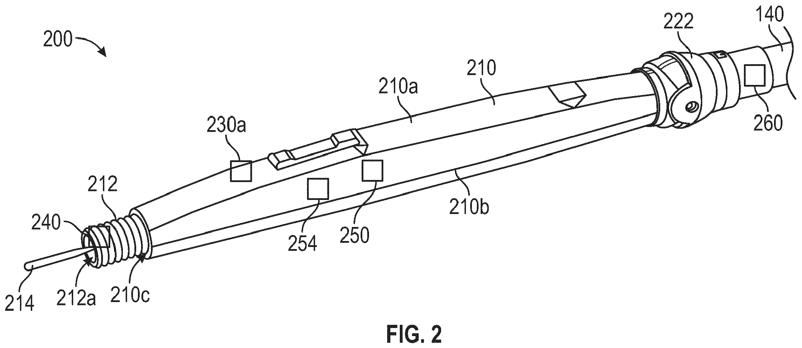

With reference to FIG. 1, a surgical smoke evacuation system 100 is shown. The surgical smoke evacuation system 100 generally includes a smoke evacuator 130, an ES pencil 200 (FIG. 2), a suction conduit 140 connecting the ES pencil 200 to the smoke evacuator 130, and one or more valves 230a-230c configured to reduce the vacuum pressure from a nozzle 212 of the ES pencil 200.

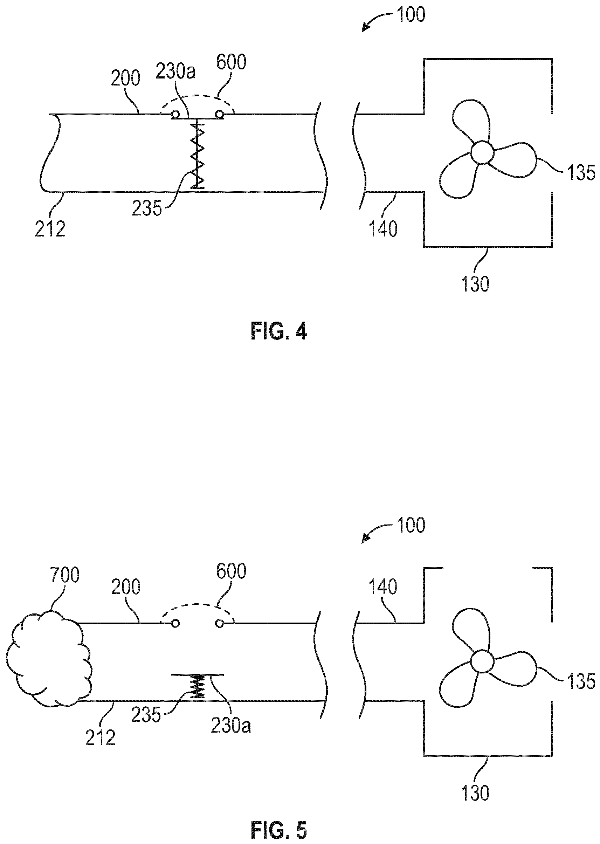

The smoke evacuator 130 includes a suction generator (e.g., suction generator 135 in FIGS. 4-8) that creates negative pressure having a set vacuum force for removing smoke during a surgical operation. The suction conduit 140 is connected to the ES pencil 200 at its distal end and to an inlet port 132 of the smoke evacuator 130 at its proximal end. The suction generator 135 may include one or more fans to create the negative pressure enabling smoke removal from a surgical site.

The valves 230a-230c are configured to reduce the vacuum pressure from a nozzle 212 of the ES pencil 200 when actuated in an open position. The valves 230a-230c may be integrated into the ES pencil 200, the suction conduit 140, and/or the smoke evacuator 130. The valves 230a-230c may be in electrical communication with a pressure sensor 240 (FIG. 2) of the ES pencil 200.

The smoke evacuator 130 also includes a processor 190 and a memory 192. Instructions may be executed by the processor 190, which may include one or more digital signal processors (DSPs), general-purpose microprocessors, application-specific integrated circuits (ASICs), field programmable logic arrays (FPGAs), or other equivalent integrated or discrete logic circuitry. Accordingly, the term "processor" as used herein may refer to any of the foregoing structures or any other physical structure suitable for implementation of the described techniques. Also, the techniques could be fully implemented in one or more circuits or logic elements. It is contemplated that the processor 190 and memory 192 may be located in the smoke evacuator 130, the ES pencil 200, and/or in a remote computer system.

The memory 192 includes instructions stored thereon which, when executed by the processor 190, cause the surgical smoke evacuation system 100 to sense the vacuum pressure at the nozzle 212, by the pressure sensor 240, and actuate the valves 230a-230c to an open position based on the sensed pressure.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

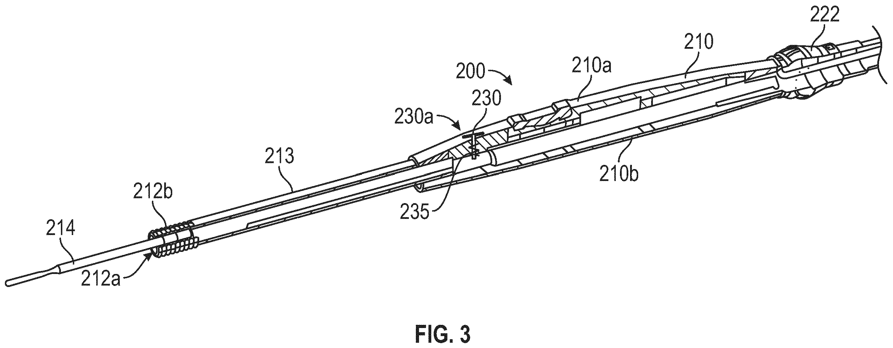

Referring to FIGS. 2 and 3, an ES pencil 200 is disclosed, which includes a handle housing 210 formed from a thermoplastic material. The handle housing 210 includes an upper portion 210a and a lower portion 210b, which are secured to each other using any suitable method, e.g., ultrasonically welding, to secure internal components of the ES pencil 200. The handle housing 10 defines a lumen 210c therethrough. The ES pencil 200 also includes a nozzle 212 that may be removably or securely coupled within the housing 210 and an electrode 214 disposed within the nozzle 212. The nozzle 212 also defines a lumen 212a, which is in fluid communication with the lumen 210c.

The nozzle 212 is formed from a dielectric material, such as polyimide, and provides for the suctioning of gaseous byproducts through the handle housing 210. In aspects, the dielectric material of the nozzle 212 may be a transparent, substantially transparent, or translucent material configured to facilitate visual acuity in the surgical field. However, it will be clear that an opaque or substantially opaque material may also be used as such materials would not affect the operation of the device. The ES pencil 200 also includes a connector 222 (e.g., a swivel connector) coupling the nozzle 212 to the suction conduit 140. As shown in FIG. 3, the nozzle 212 may also include a distal end portion 212b, which may be configured to couple to a nozzle extender attachment 213. The distal end portion 212b may have a plurality of ribs 212c for frictionally engaging the nozzle extender attachment 213. This allows the ES pencil 200 to have a longer nozzle 212 to use with a longer electrode 214 for deeper access.

The ES pencil 200 further includes a pressure sensor 240 configured to sense vacuum pressure at the nozzle 212. The pressure sensor 240 may be a piezo sensor, a cold cathode sensor, a hot cathode sensor, a capacitive sensor, a pressure switch, and/or a Pirani vacuum pressure sensor. The pressure sensor 240 senses the vacuum pressure that is used to draw smoke from the surgical site.

The ES pencil 200 may further include a valve 230a disposed on or proximate to the nozzle 212. The valve 230a is configured to reduce the vacuum pressure from a nozzle 212 when actuated in an open position. The valves 230a-230c may be used in conjunction with the pressure sensor 240 to dynamically control the vacuum pressure at the nozzle 212 of the ES pencil 200. The valves 230a-230c may be bleeder valves, flap valves, and/or any suitable type of valve. In aspects, the valves 230a-230c may have an integrated pressure sensor 240. It is contemplated that the valve 230a-230c may be mechanically designed to open at a pre-determined pressure (e.g., by selection of spring force).

The ES pencil 200 may further include a motion sensor 250 configured to sense the motion of the ES pencil 200 and activate the suction generator 135 based on the sensed motion. Smoke evacuators 130 may include a time limit control to prevent overuse of an air filter (not shown) configured to filter the air of smoke and other particulate matter. Generally, smoke evacuators 130, may include a timer that controls when suction is active, so the suction is not on continuously to increase the life span of the filter(s). The suction generator 135 and timer of the smoke evacuator 130 may be started when the ES pencil 200 is energized, i.e., in response to pressimg of an actuation pushbutton on the ES pencil 200. This may create a time lag for the full vacuum to be realized at the inlet port 132 (FIG. 1) of the suction conduit 140 due to typical length of the suction conduit 140 being 10 feet or more. This time lag may cause the user to miss the initial smoke capture.

The motion sensor 250 is in electrical communication with the processor 190 (FIG. 1). The motion sensor 250 may be an accelerometer, a gyroscope, or any other suitable sensor configured to measure movement and/or tilt of the ES pencil 200. The motion sensor 250 may be disposed on a distal end of the suction conduit 140 that turns on the suction generator 135 when the ES pencil 200 is being manipulated and a timer to turn it off when the ES pencil 200 is set down. In embodiments, the motion sensor 250 may be disposed anywhere on the ES pencil 200.

The ES pencil 200 may further include a touch sensor 254 on the ES pencil 200 configured to activate the smoke evacuator 130. The touch sensor 254 is configured to sense contact when the ES pencil 200 is grabbed by the user, indicating an intent to use the ES pencil 200. The touch sensor 254 is located on a portion of the housing 210 of the ES pencil 200 where the surgeon normally grabs the ES pencil 200 for performing surgery. The touch sensor 254 is also in electrical communication with the processor 190. By activating the smoke evacuator 130 when the ES pencil 200 is being manipulated, the surgical smoke evacuation system 100 can capture more smoke than activating the smoke evacuator 130 only when the ES pencil 200 is electrically active.

The connector 222 of the ES pencil 200 may further be configured for electrical communication between the pressure sensor and the suction source (e.g., smoke evacuator 130) to prevent a standard suction conduit from being used with the surgical smoke evacuation system 100 of FIG. 1. This connector 222 may include a mating sensor 260 configured to sense a mating connection with the suction generator 135. The mating sensor 260 may include a unique identifier, which may be used by the processor to detect and confirm if the ES pencil 200 is mated with the correct suction generator 135. The mating sensor 260 may include a Radio Frequency Identification (RFID) sensor configured to send and receive the unique identifier to the processor 190 to enable mating of the ES pencil 200 with the smoke evacuator 130. The smoke evacuator 130 may include an RFID tag (not shown) configured to be scanned by the RFID reader of the mating sensor 260 of the ES pencil 200.

The connector 222 further may include a mechanical interface configured to mate with a suction conduit 140 having a counterpart mechanical interface. For example, the mechanical interface may include an outer shape such as a triangular shape, or other suitable outer shape. Thus, if the connector was the wrong outer shape, e.g., round instead of triangular, or the RFID indicated the wrong unique identifier, the suction generator 135 of the smoke evacuator 130 may be limited to generating a lower vacuum (e.g., below about -15 kPa).

FIGS. 4 and 5 illustrate a side cross-sectional view of the surgical smoke evacuation system 100. As shown in FIGS. 4 and 5, the ES pencil 200 may further include a cover 600 to prevent occlusion of the valve 230a when the valve 230a is in an open position. The valve cover 600 may be a mesh cover, or any other cover which permits the flow of air. For example, the cover 600 may include a number of (e.g., two) inlets located a distance apart such that both inlets would not be easily covered, for example, by the pad of a single finger. The cover may include raised ridges with inlet holes in troughs would allow airflow even when covered, for example by by a finger.

In an aspect, the processor 190 may control higher vacuum pressure with the pressure sensor 240 at the nozzle 212 (FIG. 2) by enabling dynamic control of the vacuum pressure generated by the suction generator 135 of the smoke evacuator 130. If the nozzle 212 is occluded (e.g., by tissue 700 as in FIG. 5), then the processor 190 (FIG. 1) may sense a vacuum at the nozzle 212 above a threshold, which may be about -15 kPa, and may control the amount of vacuum generated by the suction generator 135 to equalize the pressure to a maximum of about - 15 kPa at the inlet port 132 to the smoke evacuator 130. Even though -15 kPa is used as an example threshold, other thresholds are contemplated by the disclosure.

In another example, the suction generator 135 may generate a vacuum pressure at inlet port 132 of the smoke evacuator 130 to be above a certain evacuator threshold, which may be about -60 kPa to compensate for pressure drop at the ES pencil 200. The vacuum pressure may drop along the suction conduit 140 and the length of the ES pencil 200. This reduced vacuum pressure may be sensed by the pressure sensor 240 at the nozzle 212 at a lower value, for example, about -13 kPa. In this example, the nozzle 212 may be partially occluded (e.g., by tissue 700), causing the vacuum to increase up to about -15 kPa. The processor 190 (FIG. 1) receives a signal from the pressure sensor 240 (FIG. 2), which is located at the nozzle 212, compares the sensed vacuum pressure to the predetermined nozzle threshold (about -15 kPa), and control the smoke evacuator 130 to reduce the vacuum pressure.

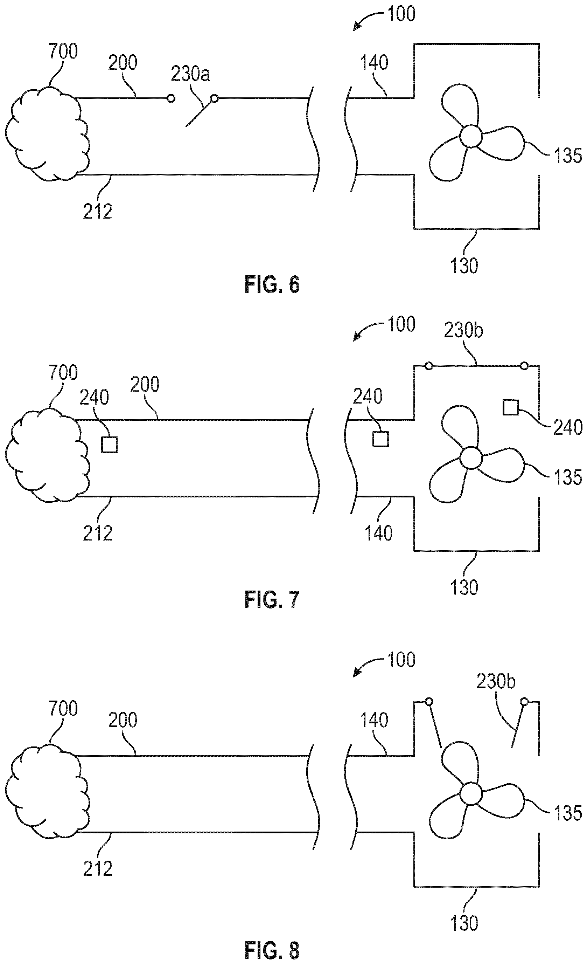

FIG. 6 shows a side, cross-sectional view of the surgical smoke evacuation system of FIG. 1 with the valve 230a shown as a flapper bleed valve. In a case where the nozzle 212 is occluded (e.g., by tissue), the valve 230a occludes the main smoke flow until the tissue (or other occlusion) is removed from the nozzle 212. The suction generator 135 may include active vacuum control, controlled by processor 190. For example, as the nozzle 212 is occluded, the pressure sensor 240 (FIG. 2) senses an increase in pressure above a predetermined nozzle threshold (e.g., about -15 kPa), and the processor 190 reduces the amount of vacuum generated by the suction generator 135 of the smoke evacuator 130.

FIGS. 7 and 8 illustrate side, cross-sectional side views of the surgical smoke evacuation system of FIG. 1, with the valve 230b located at a smoke evacuator 130 of the surgical smoke evacuation system of FIG. 1. For example, processor 190 can sense, by the pressure sensor 240, that the vacuum pressure at the nozzle 212 of the ES pencil 200 is about -60 kPa (because the nozzle 212 is occluded, for example, by tissue 700). The processor 190 may actuate the valve 230b located near the suction generator 135 to the open position to quickly reduce the vacuum from about -60 kPa to about -15 kPa. It is contemplated that additional pressure sensors 240 along suction conduit 140 and within the smoke evacuator 130 may also be used to regulate the vacuum pressure (FIG. 7).

It will be understood that various modifications may be made to the aspects of the presently disclosed smoke evacuation ES pencils. Therefore, the above description should not be construed as limiting but merely as exemplifications of aspects. Those skilled in the art will envision other modifications within the scope and spirit of the disclosure.

The invention may be described by reference to the following numbered paragraphs:-
1. An electrosurgical pencil comprising:
   a handle housing having a proximal end portion and a distal end portion, the handle housing defining a lumen therethrough;
   a nozzle in fluid communication with the lumen;
   a pressure sensor configured to sense a vacuum pressure at the nozzle;
   a connector coupled to the distal end portion of the handle housing, the connector configured to couple to a suction source; and
   an electrode configured to couple to a source of electrosurgical energy.
2. The electrosurgical pencil according to paragraph 1, further comprising a valve configured to reduce or remove the vacuum pressure at the nozzle when actuated.
3. The electrosurgical pencil according to paragraph 2, wherein the valve is configured to actuate to an open position in response to a pressure signal measured by the pressure sensor exceeding a preset threshold.
4. The electrosurgical pencil according to paragraph 3, wherein the pressure sensor is integrated into the valve.
5. The electrosurgical pencil according to paragraph 1, further comprising a motion sensor disposed on the nozzle, wherein the motion sensor is configured to transmit a motion signal to the suction source to initiate suction.
6. The electrosurgical pencil according to paragraph 2, wherein the valve includes a cover configured to prevent actuation of the valve by an external force.
7. The electrosurgical pencil according to paragraph 1, wherein the connector includes a mating sensor configured to sense a mating connection with the suction source.
8. The electrosurgical pencil according to paragraph 7, wherein the mating sensor includes a unique identifier.
9. The electrosurgical pencil according to paragraph 1, wherein the connector is further configured for electrical communication between the pressure sensor and the suction source.
10. The electrosurgical pencil according to paragraph 1, wherein the connector includes a mechanical interface configured to mate with a suction conduit having a counterpart mechanical interface.
11. A surgical smoke evacuation system, comprising:
   a smoke evacuator including a suction generator configured to create a vacuum pressure;
   an electrosurgical pencil including:
      a nozzle; and
      a pressure sensor configured to sense vacuum pressure at the nozzle;
   a suction conduit coupling the nozzle to the smoke evacuator;
   a valve configured to reduce the vacuum pressure from the nozzle;
   a processor; and
   a memory, including instructions stored thereon which, when executed by the processor, cause the surgical smoke evacuation system to:
      sense the vacuum pressure at the nozzle; and
      actuate the valve to an open position based on the sensed pressure.
12. The smoke evacuation system according to paragraph 11, wherein the valve is configured to open to reduce the vacuum pressure from the nozzle of the electrosurgical pencil in response to the sensed vacuum force being greater than a predetermined threshold.
13. The smoke evacuation system according to paragraph 11, further comprising a motion sensor disposed on the nozzle,
   wherein the instructions, when executed by the processor, further causes the system to:
   sense a signal by the motion sensor indicating movement of the electrosurgical pencil; and
   initiate suction by the smoke evacuator based on the sensed signal.
14. The smoke evacuation system according to paragraph 11, further comprising a motion sensor disposed on the nozzle,
   wherein the instructions, when executed by the processor, further causes the system to:
   sense a signal by the motion sensor indicating that the electrosurgical pencil is at a resting position; and
   stop suction by the smoke evacuator based on the sensed signal.
15. The smoke evacuation system according to paragraph 11, wherein the valve includes a cover configured to prevent actuation of the valve by an external force.
16. The smoke evacuation system according to paragraph 11, wherein the pressure sensor is integrated into the valve.
17. The smoke evacuation system according to paragraph 11, wherein the instructions, when executed by the processor, further cause the system to:
   dynamically adjust the vacuum pressure generated by the smoke evacuator based on the sensed vacuum pressure at the nozzle.
18. The smoke evacuation system according to paragraph 11, wherein the instructions, when executed by the processor, further cause the system to:
   determine the nozzle is occluded based on the sensed signal; and
   dynamically adjust the vacuum pressure generated by the smoke evacuator based on the determined occlusion.
19. The smoke evacuation system according to paragraph 18, wherein the instructions, when executed by the processor, further cause the system to:
   determine whether occlusion is removed based on the sensed signal; and
   dynamically increase the vacuum pressure generated by the smoke evacuator based on the determination the occlusion was removed.
20. The smoke evacuation system according to paragraph 11, wherein the electrosurgical pencil includes a connector configured to couple to a suction source, wherein the connector includes a mating sensor configured to sense a mating connection with the suction source.

## Claims

1. An electrosurgical pencil comprising:
a handle housing having a proximal end portion and a distal end portion, the handle housing defining a lumen therethrough;
a nozzle in fluid communication with the lumen;
a pressure sensor configured to sense a vacuum pressure at the nozzle;
a connector coupled to the distal end portion of the handle housing, the connector configured to couple to a suction source; and
an electrode configured to couple to a source of electrosurgical energy.

2. The electrosurgical pencil according to claim 1, further comprising a valve configured to reduce or remove the vacuum pressure at the nozzle when actuated, preferably wherein the valve includes a cover configured to prevent actuation of the valve by an external force.

3. The electrosurgical pencil according to claim 2, wherein the valve is configured to actuate to an open position in response to a pressure signal measured by the pressure sensor exceeding a preset threshold preferably, wherein the pressure sensor is integrated into the valve.

4. The electrosurgical pencil according to any one of claims 1 to 3, further comprising a motion sensor disposed on the nozzle, wherein the motion sensor is configured to transmit a motion signal to the suction source to initiate suction.

5. The electrosurgical pencil according to any preceding claim, wherein the connector includes a mating sensor configured to sense a mating connection with the suction source, preferably wherein the mating sensor includes a unique identifier.

6. The electrosurgical pencil according to any preceding claim, wherein the connector is further configured for electrical communication between the pressure sensor and the suction source.

7. The electrosurgical pencil according to any preceding claim, wherein the connector includes a mechanical interface configured to mate with a suction conduit having a counterpart mechanical interface.

8. A surgical smoke evacuation system, comprising:
a smoke evacuator including a suction generator configured to create a vacuum pressure;
an electrosurgical pencil including:
a nozzle; and
a pressure sensor configured to sense vacuum pressure at the nozzle;
a suction conduit coupling the nozzle to the smoke evacuator;
a valve configured to reduce the vacuum pressure from the nozzle;
a processor; and
a memory, including instructions stored thereon which, when executed by the processor, cause the surgical smoke evacuation system to:
sense the vacuum pressure at the nozzle; and
actuate the valve to an open position based on the sensed pressure.

9. The smoke evacuation system according to claim 8, wherein the valve is configured to open to reduce the vacuum pressure from the nozzle of the electrosurgical pencil in response to the sensed vacuum force being greater than a predetermined threshold.

10. The smoke evacuation system according to claim 8 or 9, further comprising a motion sensor disposed on the nozzle,
wherein the instructions, when executed by the processor, further causes the system to:
sense a signal by the motion sensor indicating movement of the electrosurgical pencil; and
initiate suction by the smoke evacuator based on the sensed signal.

11. The smoke evacuation system according to claim 8, 9 or 10 further comprising a motion sensor disposed on the nozzle,
wherein the instructions, when executed by the processor, further causes the system to:
sense a signal by the motion sensor indicating that the electrosurgical pencil is at a resting position; and
stop suction by the smoke evacuator based on the sensed signal.

12. The smoke evacuation system according to any one of claims 8 to 11, wherein the valve includes a cover configured to prevent actuation of the valve by an external force.

13. The smoke evacuation system according to any one of claims 8 to 12, wherein the pressure sensor is integrated into the valve.

14. The smoke evacuation system according to any one of claims 8 to 13 , wherein the instructions, when executed by the processor, further cause the system to:
dynamically adjust the vacuum pressure generated by the smoke evacuator based on the sensed vacuum pressure at the nozzle, preferably wherein the instructions, when executed by the processor, further cause the system to:
determine the nozzle is occluded based on the sensed signal; and
dynamically adjust the vacuum pressure generated by the smoke evacuator based on the determined occlusion further preferably wherein the instructions, when executed by the processor, further cause the system to:
determine whether occlusion is removed based on the sensed signal; and
dynamically increase the vacuum pressure generated by the smoke evacuator based on the determination the occlusion was removed.

15. The smoke evacuation system according to any one of claims 8 to 14, wherein the electrosurgical pencil includes a connector configured to couple to a suction source, wherein the connector includes a mating sensor configured to sense a mating connection with the suction source.
